# EUROPÄISCHE PATENTANMELDUNG

(11) **EP 2 394 630 A2**
(43) Veröffentlichungstag der Anmeldung: **14.12.2011**
(21) Anmeldenummer: 11167650.8
(22) Anmeldetag: 26.05.2011
(51) Int. Cl.: A61K 8/14, A61K 8/41, A61Q 5/06

(54) **Kosmetische Zusammensetzung zum Färben der Haare mit einem direktziehenden Haarfarbstoff und mit einem Trägersystem aus Lipidvesikeln**

(30) Priorität: 11.06.2010 DE 102010030001
(71) Anmelder: ROVI Cosmetics International GmbH, 36381 Schlüchtern (DE)
(72) Erfinder: Beyer, Monika, 61130 Nidderau-Ostheim (DE); Teichmüller, Dirk, 63589 Linsengericht (DE)
(74) Vertreter: WSL Patentanwälte

(57) **Zusammenfassung**

Die vorliegende Erfindung betrifft kosmetische Zusammensetzungen zum Färben der Haare mit wenigstens einem direktziehenden Haarfarbstoff und mit einem Trägersystem für den wenigstens einen direktziehenden Haarfarbstoff, die Verwendung solcher Zusammensetzungen in kosmetischen Formulierungen zum Färben der Haare sowie Verfahren zur Herstellung der Zusammensetzungen. Um eine Möglichkeit bereitzustellen, mit der die Dauerhaftigkeit der Bindung von direktziehenden Haarfarbstoffen an das Haar verbessert werden kann mit dem Ziel, dass direktziehende Farbstoffe möglichst lang am Haar verbleiben, um die gewünschte Haarfarbe möglichst lang in der gewünschten Qualität zu liefern, werden erfindungsgemäß Zusammensetzungen der vorgenannten Art vorgeschlagen, bei denen das Trägersystem Lipidvesikel mit einer oder mehreren Lipidmembran(en) umfasst, wobei der wenigstens eine direktziehende Haarfarbstoff in den Lipidvesikeln enthalten ist und die Lipidvesikel eine positive Oberflächenladung aufweisen, wobei die positive Oberflächenladung dadurch bewirkt wird, dass die Lipidvesikel in der/den Lipidmembran/en zusätzlich zu den Lipiden, aus denen die Vesikel aufgebaut sind, positiv geladene Alkyl-Trimoniumsalze als Ladungsgeber aufweisen.

## Beschreibung

Die vorliegende Erfindung betrifft kosmetische Zusammensetzungen zum Färben der Haare mit wenigstens einem direktziehenden Haarfarbstoff und mit einem Trägersystem für diesen wenigstens einen direktziehenden Haarfarbstoff, die Verwendung solcher Zusammensetzungen in kosmetischen Formulierungen zum Färben der Haare sowie ein Verfahren zur Herstellung der Zusammensetzungen.

Viele Menschen wünschen sich vorübergehend oder dauerhaft eine andere als ihre natürliche Haarfarbe, und es gibt eine ganze Reihe von Verfahren, um seinem Haar eine andere Farbe zu verleihen.

Bei einem dieser Verfahren werden direktziehende Haarfarbstoffe verwendet, die sich an der Cuticula des Haares anlagern oder sich darin einlagern. Bei diesem semi-permanenten oder temporären Verfahren handelt sich damit um eine Färbung, bei der das natürlich vorhandene Pigment des Haares nicht verändert wird, so dass sich zusammen mit der natürlichen Haarfarbe der neue Farbton ergibt. Im Gegensatz dazu gibt es auch Verfahren mit Haarfarbstoffen, die die natürliche Haarfarbe chemisch verändern, indem Sie bspw. chemisch an bestimmte Aminosäuren im Haar binden.

Viele direktziehende Haarfarbstoffe lagern sich von Hause aus nicht mit der gewünschten Dauerhaftigkeit an die Cuticula an bzw. in die Cuticula ein und manche dringen - beispielsweise wegen Ihrer Molekülgröße - überhaupt nicht bis in den Cortex des Haares vor. Haarfärbemittel mit solchen Haarfarbstoffen führen daher oft nicht zu einer Färbung mit der gewünschten Dauerhaftigkeit. Es besteht daher ein Bedarf nach einer Möglichkeit, die Dauerhaftigkeit der An- bzw. Einlagerung von direktziehenden Haarfarbstoffen zu verlängern.

Der vorliegenden Erfindung liegt daher die Aufgabe zugrunde, eine Möglichkeit bereitzustellen, mit der die Dauerhaftigkeit der Bindung von direktziehenden Haarfarbstoffen an bzw. in das Haar verbessert werden kann. Ziel ist es, dass erreicht wird, dass direktziehende Farbstoffe möglichst lang am Haar verbleiben, um die gewünschte Haarfarbe möglichst lang in der gewünschten Qualität zu liefern.

Diese Aufgabe wird erfindungsgemäß gelöst durch Zusammensetzungen, mit wenigstens einem direktziehenden Haarfarbstoff und mit einem Trägersystem für den wenigstens einen direktziehenden Haarfarbstoff, wobei das Trägersystem Lipidvesikel mit einer oder mehreren Lipidmembran(en) umfasst, dadurch gekennzeichnet, dass der wenigstens eine direktziehende Haarfarbstoff in den Lipidvesikeln enthalten ist und die Lipidvesikel eine positive Oberflächenladung aufweisen, wobei die positive Oberflächenladung dadurch bewirkt wird, dass die Lipidvesikel in der/den Lipidmembran/en zusätzlich zu den Lipiden, aus denen die Vesikel aufgebaut sind, positiv geladene quartäre Ammoniumverbindungen als Ladungsgeber aufweisen, wobei diese Ladungsgeber aus Alkyl-Trimoniumsalzen der Formel 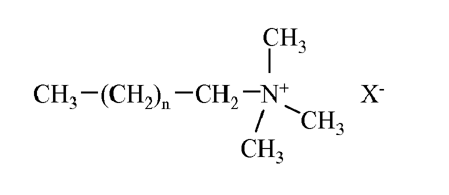 ausgewählt sind, wobei
n eine Zahl von 18 bis 28 und
X- ein anorganisches oder organisches Anion ist.

Direktziehende Farbstoffe liefern vermittelt durch das Trägersystem der erfindungsgemäßen Zusammensetzung die gewünschte Haarfarbe länger in der gewünschten Qualität als ohne das Trägersystem der erfindungsgemäßen Zusammensetzung, was wohl darauf zurückzuführen sein wird, dass direktziehende Farbstoffe vermittelt durch das Trägersystem der erfindungsgemäßen Zusammensetzung länger auf oder in dem Haar verbleiben.

Vermutlich wird durch das Trägersystem der erfindungsgemäßen Zusammensetzung erreicht, dass die Dauerhaftigkeit der An- bzw. Einlagerung von direktziehenden Haarfarbstoffen an bzw. in das Haar verbessert wird, indem der Farbstoff fester auf dem Haar anhaftet bzw. besser in das Haar penetriert, um dort gezielt freigesetzt und eingelagert zu werden, wobei diese Feststellungen in Bezug auf die vorliegende Erfindung jedoch nicht bindend sein sollen, und der Umfang der Erfindung durch diese Feststellungen auch nicht beschränkt werden soll.

Zusätzlich bewirkt die erfindungsgemäße Zusammensetzung eine bessere Stabilisierung des Haarfarbstoffs, was zu einer Erhöhung der Menge an intaktem Haarfarbstoff, die an oder in den Haaren bereitgestellt wird, führt, was wiederum den Effekt hat, dass die gewünschte Haarfarbe länger in der gewünschten Qualität aufrechterhalten werden kann. Die positive Ladung der Vesikeloberfläche wird erreicht, indem die Lipidvesikel zusätzlich zu den Lipiden, aus denen die Vesikel aufgebaut sind, positiv geladene Moleküle als Ladungsgeber umfassen.

Erfindungsgemäß handelt es sich bei den positiv geladenen Molekülen bzw. Ladungsgebern um Alkyl-Trimoniumsalze (bzw. Fettsäure-Trimoniumsalze) der Formel 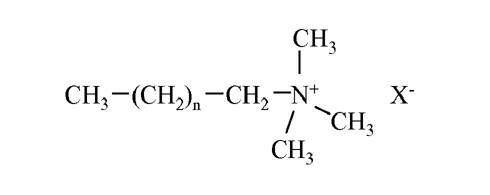 wobei n eine ganze Zahl von 18 bis 28 und X- ein anorganisches oder organisches Anion ist.

Vorzugsweise ist X- ein Halogenid-Ion oder das Anion einer organischen Säure, die ausgewählt ist unter einer kosmetisch verträglichen Carbonsäure oder Sulfonsäure. Besonders bevorzugt ist X⁻ Bromid, Chlorid, Fluorid, Iodid, Saccharinat, Tosylat oder Methosulfat.

Vorzugsweise ist n in der oben angegebenen Alkyl-Trimoniumsalzformel gleich 22. Inbesondere bevorzugt ist das Alkyl-Trimoniumsalz Behentrimoniumchlorid.

Vorzugweise werden die positiv geladenen Ladungsgeber in einer Menge von 0,01 bis 10 Gew.-%, bevorzugt 0,01 bis 2,0 Gew.-%, bezogen auf die gesamte Zusammensetzung eingesetzt.

Vorzugsweise haben die Lipidvesikel ein Zetapotential im Bereich von 1 bis 200 mV. Der Begriff "Zetapotential" beschreibt das elektrische Potential einer Abscherschicht eines bewegten Partikels in einer Suspension. Die Messung des Zetapotentials kann dadurch erfolgen, dass man Partikel durch ein angelegtes elektrisches Feld bewegt. Aus der resultierenden Geschwindigkeit der Partikel lässt sich dann das Zetapotential berechnen.

Das Zetapotential wird im Zusammenhang mit der vorliegenden Erfindung mittels Laser-Doppler-Elektrophorese bestimmt. Dabei erfolgen die Messungen in stark verdünnten wässrigen Kochsalzlösungen, wobei die zu vermessenden Proben üblicherweise in Konzentrationen von 0,01-0,1 Gew.-% in 1 mM Natriumchloridlösung vorliegen. Die pH-Werte der Probenlösungen liegen im pH-Wert-Spezifikationsbereich der jeweiligen zu vermessenden Produkte (erfindungsgemäße UV-Schutzmittel und Formulierungen), wobei im Regelfall produktspezifisch pH-Werte im Bereich von pH 5,5 bis maximal pH 7,5 getroffen werden.

Bei bestimmten Ausführungsformen haben die Lipidvesikel ein Zetapotential im Bereich von 1 bis 150 mV. Bei manchen Ausführungsformen weisen die Vesikel ein Zetapotential von 30 bis 100 mV auf. Bei besonders bevorzugten Lipidvesikeln beträgt das Zetapotential 40 bis 60 mV.

Die Teilchengröße der erfindungsgemäßen Lipidvesikel ist vorzugsweise von 50 bis 1000 nm. Bei bestimmten Ausführungsformen ist die Teilchengrlöße in dem Bereich 100 bis 400 nm oder in dem Bereich 100 bis 350 nm oder in dem Bereich 100 bis 250 nm.

Die erfindungsgemäße Zusammensetzung kann Vesikel mit einer Lipidmembran (Nanosomen), zwei Lipidmembranen (Liposomen) oder mehreren Lipidmembranen umfassen. Dadurch wird eine Anpassung an verschiedene Verwendungen der Zusammensetzung ermöglicht.

Die Lipide sind bevorzugt unter Ceramiden, Phospholipiden, Glycosphingolipiden und/oder Di-Acylglycosiden ausgewählt. Dazu gehören auch Sphingomyeline, Galactocerebroside und Glucocerebroside, Dihexoside, Tri- und Tetrahexoside sowie Ganglioside. Die Phospholipide, die bei der Zusammensetzung für die Bildung der Vesikel verwendet werden können, können unter allen kosmetisch verträglichen Phospholipiden ausgewählt werden, die in der Lage sind, in wässrigem Milieu Vesikel (Nanosomen oder Liposomen) zu bilden. Bevorzugte Phospholipide sind Lecithin, Phosphatidylcholin, Phosphatidylethanolamin und Phosphatidylserin.

Bei speziellen Ausführungsformen können auch Gemische der oben genannten Lipide eingesetzt werden. Der Anteil der Lipide bezogen auf die gesamte Zusammensetzung beträgt vorzugsweise 1 bis 20 Gew.-% oder 1 bis 15 Gew.-% oder 4 bis 10 Gew.-%.

Als direktziehende Haarfarbstoffe kommen bei der vorliegenden Erfindung uneingeschränkt alle alle laut Kosmetikverordnung gegenwärtig zugelassenen und künftig zugelassenen wasser- und fettlöslichen Haarfarbstoffe sowie beliebige Kombinationen dieser Farbstoffe in Betracht, die verkapselt werden können. Hierbei kann es sich sowohl um lipophile (öllösliche) als auch um hydrophile (wasserlösliche) Farbstoffe handeln, die aus natürlichen Quellen isoliert oder chemisch oder biotechnologisch hergestellt wurden, wie z.B. Pflanzenfarbstoffe wie Henna oder Indigo, Inoaniline, Indophenole, Nitrobenzolderviate, Anthrachinon-Farbstoffe, Triphenylmethanfarbstoffe, Chinonfarbstoffe, Azofarbstoffe, kationische und anionische Farbstoffe.

Ein Haarfarbstoff ist im Sinne der vorliegenden Erfindung dann lipophil, wenn er bei 25°C einen *n*-Oktanol-Wasser-Verteilungskoeffizient *K*_{ow} aufweist, der größer als 1 ist. Ein Haarfarbstoff ist im Sinne der vorliegenden Erfindung dann hydrophil, wenn er bei 25°C einen n-Oktanol-Wasser-Verteilungskoeffizient *K*_{ow} aufweist, der kleiner oder gleich 1 ist.

Beispiele für direktziehende Haarfarbstoffe, die bei der vorliegenden Erfindung zur Anwendung kommen können, sind ohne Beschränkung hierauf:

**Tabelle 1: direktziehende Haarbfarbstoffe**

| **Coll-pa No.** | **INCI** | **CAS-Nr.** | **EINECS-Nr.** | **Color-Index-Nr** |
|---|---|---|---|---|
| B001 | Acid Yellow 1 | | | |
| B005 | Disperse Red 17 | 3179-89-3 | 221-665-5 | CI 11210 |
| B007 | Basic Brown 17 | 68391-32-2 | 269-944-0 | CI 12251 |
| B024 | 4-NITRO-o-PHENYLENEDIAMINE | 99-56-9 | 202-766-3 | CI 76020 |
| B031 | HC RED NO. 13 | 29705-39-3 | 94158-13-1 | |
| B034 | N,N'-BIS(2-HYDROXYETHYL)-2-NITRO-p-PHENYLENEDIAMINE | 84041-77-0 | 281-856-4 | |
| B036 | HC RED NO. 7 | 24905-87-1 | 246-521-9 | |
| B037 | HC BLUE NO. 2 | 33229-34-4 | 251-410-3 | |
| B047 | HC ORANGE NO. 1 | 54381-08-7 | 259-132-4 | |
| B048 | HC RED NO. 1 | 2784-89-6 | 220-494-3 | |
| B051 | 4-AMINO-3-NITROPHENOL | 610-81-1 | 210-236-8 | |
| B052 | 2-HYDROXYETHYLAMINO-5-NITROANISOLE | 66095-81-6 | 266-138-0 | |
| B054 | 3-NITRO-p-HYDROXYETHYLAMINOPHENOL | 65235-31-6 | 265-648-0 | |
| B055 | 2-AMINO-3-NITROPHENOL | 603-85-0 | | |
| B056 | 6-NITRO-o-TOLUIDINE | 570-24-1 | 209-329-3 | |
| B058 | 3-METHYLAMINO-4-NITROPHENOXY-ETHANOL | 59820-63-2 | 261-940-7 | |
| B060 | 2-NITRO-5-GLYCERYL METHYLANI-LINE | 80062-31-3 | 279-383-3 | |
| B063 | HC YELLOW NO. 11 | 73388-54-2 | - | |
| B066 | HC VIOLET NO. 1 | 82576-75-8 | 417-600-7 | |
| B067 | HC ORANGE NO. 2 | 85765-48-6 | 416-410-1 | |
| B069 | HC YELLOW NO. 9 | 141973-33-3 | 86419-69-4 | |
| B070 | 4-NITROPHENYL AMINOETHYLU-REA | 27080-42-8 | 410-700-1 | |
| B071 | HC RED NO. 10 AND HC RED NO. 11 | 95576-89-9 + 95576-92-4 | 408-240-1 | |
| B072 | 2-HYDROXYETHYL PICRAMIC ACID | 99610-72-7 | 412-520-9 | |
| B073 | HC BLUE NO. 12 | 132885-85-9 | 407-020-2 | |
| B075 | HYDROXYETHYL-2-NITRO-P-TOLUIDINE | 100418-33-5 | 408-090-7 (ELINCS) | |
| B077 | HC BLUE NO. 11 | 23920-15-2 | 459-980-7 | |
| B080 | HC YELLOW NO. 7 | 104226-21-3 | | |
| B087 | 4-AMINO-2-NITROPHENYL-AMINE-2'-CARBOXYLIC ACID | 117907-43-4 | - | |
| B089 | 2-CHLORO-6-ETHYLAMINO-4-NITROPHENOL | 131657-78-8 | 411-440-1 | |
| B098 | HC VIOLET NO. 2 | | | |
| B099 | 2-Amino-6-Chloro-4-Nitrophenol | 6358-09-4 | 228-762-1 | |
| B100 | 4-Hydroxypropylamino-3-Nitrophenol | 92952-81-3 | 406-305-9 | |
| B102 | HC YELLOW NO. 13 | 10442-83-8 | | |
| B104 | 1,2,3,4-TETRAHYDRO-6-NITROCHINOXALIN | 158006-54-3 (hydrochloride) | - | |
| B113 | Basic Orange No.69 | 226940-14-3 | - | CI 112605 |
| B115 | Basic Violet 2 | 3248-91-7 | 221-831-7 | CI 42520 |
| C008 | Basic Red 76 | 68391-30-0 | 269-941-4 | CI 12245 |
| C009 | Basic Brown 16 | 26381-41-9 | 247-640-9 | CI 12250 |
| C010 | Basic Yellow 57 | 68391-31-1 | 269-943-5 | CI 12719 |
| C015 | Orange 4 | 633-96-5 | 211-199-0 | CI 15510 |
| C022 | Red 33 | 3567-66-6 | 222-656-9 | CI 17200 |
| C029 | Yellow 5 - Acid Yellow 23 | 1934-21-0 | 217-699-5 | CI 19140 |
| C040 | Acid Blue 9 - Blue 1 | 3844-45-9 | 4223-333-98 | CI 42090 |
| C046 | Basic Blue 7 | 2390-60-5 | | CI 42595 |
| C053 | Acid Red 92 | 18472-87-2 | 242-355-6 | CI 45410 |
| C054 | Yellow 10 - Acid Yellow 3 | - | - | CI 47005 |
| C059 | Basic Blue 99 | 68123-13-7 | - | CI 56059 |
| C063 | Acid Violet 43 - Ext. Violet 2 | 4430-18-6 | 224-618-7 | CI 60730 |
| C064 | Disperse Violet 1 | 128-95-0+116-85-8 | - | |
| C067 | ACID BLUE 62 | 4368-56-3 | 224-460-9 | CI 62045 |
| C117 | HYDROXYANTHRAQUINONEAMI-NOPROPYLMETHYL MORPHOLINIUM METHOSULFATE | 38866-20-5 | 254-161-9 | |
| C119 | HC RED NO. 8 | 13556-29-1 | 306-778-0 | |
| C129 | HC GREEN NO. 1 | 52136-25-1 | 257-687-7 | |
| C146 | LAWSONE | 83-72-7 | - | CI 75480 |
| C169 | Henna (Lawsonia inermis) | 84988-66-9 | - | |
| C172 | HC BLUE No. 14 | 99788-75-7 | - | |
| C174 | CURRY RED | 25956-17-6 | 247-368-0 | CI 16035 |
| C175 | Acid Red 18 | 2611-82-7 | 220-036-2 | CI 16255 |
| C177 | Acid Red 52 | 3520-42-1 | 222-529-8 | CI 45100 |
| C178 | Acid Green 25 | 4403-90-1 | 224-546-6 | CI 61570 |

Vorzugsweise beträgt der Anteil an direktziehendem Haarfarbstoff bei der vorliegenden Erfindung bezogen auf die gesamte Zusammensetzung 0,01 bis 40 Gew.-%.

Bei bestimmten Ausführungsformen liegen Kombinationen aus zwei oder mehreren direktziehenden Haarfarbstoffen vor, wobei die Summe dieser Farbstoffe bezogen auf die gesamte Zusammensetzung 0,01 bis 40 Gew.-% ausmacht.

In einer Ausführungsform der vorliegenden Erfindung ist der wenigstens eine direktziehende Haarfarbstofff in der/den Lipidmembran(en) enthalten, d.h. zwischen den Lipiden der Lipidmembran in die Lipidmembran eingebunden. Vorzugsweise beträgt der Anteil an Haarfarbstoff bei diesen Ausführungsformen bezogen auf die gesamte Zusammensetzung 0,01 bis 15 Gew.-%. Handelt es sich bei dem wenigstens eine Haarfarbstoff allerdings um einen lipophilen Haarfarbstoff, beträgt der Anteil dieses Haarfarbstoffs bezogen auf die gesamte Zusammensetzung vorzugsweise 0,01 bis 10 Gew.-%.

In einer anderen Ausführungsform ist der wenigstens eine direktziehende Haarfarbstoff von der/den Lipidmembran(en) umkapselt. Der Begriff "umkapselt" ist hier so zu verstehen, dass der Haarfarbstoff in einer Flüssigkeit, die sich in dem von einer Lipidmembran gebildeten Vesikelinnenraum befindet, gelöst vorliegt. Bei einer bevorzugten Ausführungsform liegt der Haarfarbstoff in einer im Vesikelinneren eingeschlossenen Wasserphase gelöst vor. Vorzugsweise beträgt der Anteil an Haarfarbstoff bei diesen Ausführungsformen bezogen auf die gesamte Zusammensetzung 5 bis 40 Gew.-%.

Es hat sich gezeigt, dass direktziehende Haarfarbstoffe, wenn sie im Vesikelinnenraum eingeschlossen vorliegen, bzw. wenn sie in der Lipidmembran eines Vesikels enthalten sind, zu einer Verbesserung der Haltbarkeit der Haarfarbstoffe auf der Haaroberfläche führen.

Erfindungsgemäß wird die Zusammensetzung in kosmetischen Formulierungen zum Färben der Haare verwendet. Dabei können der Zusammensetzung, die positiv geladene Lipidvesikel und wenigstens einen direktziehenden Haarfarbstoff umfasst, weitere Haarfarbstoffe hinzugefügt werden, die entweder in den Lipidvesikeln oder außerhalb der Lipidvesikel und in der übrigen Substanz bzw. Trägermatrix der Formulierung enthalten sind.

Der Anteil der Farbstoffe an der Gesamtformulierung beträgt vorzugsweise 0,001 - 40 %.

Die Formulierungen zum Färben der Haare können alle Hilfs- und Zusatzstoffe, die üblicherweise bei kosmetischen Präparaten Anwendung finden, enthalten. Insbesondere fallen unter den Begriff "Hilfsstoff" im Zusammenhang mit der vorliegenden Erfindung solche Zusatzstoffe, die auf die physikalischen Eigenschaften der Vesikel und deren Stabilität einwirken und/oder der Konservierung der Zusammensetzung dienen. Beispiele für solche Hilfsstoffe sind Öle, Alkohole, Polyole, Antioxidantien, Gelbildner, Puffer, Konservierungsmittel, Bakterizide und Keimhemmer, Verdicker oder Komplexbildner.

Die erfindungsgemäße Zusammensetzung kann in allen zum Färben der Haare geeigneten Formulierungen vorliegen, beispielsweise in Form eines farbgebenden Shampoos oder farbgebenden Schaumfestigers oder in Form von Tönungslotionen, Schaumtönungen oder als Tönungsmousse.

Selbstverständlich handelt es sich bei allen Komponenten der erfindungsgemäßen Zusammensetzungen und Formulierungen um kosmetisch verträgliche Stoffe. Ein Stoff ist im Sinne dieser

Erfindung kosmetisch verträglich, wenn er nicht toxisch ist und bei der Mehrzahl der potentiellen Anwender anwendbar ist, ohne dass es bei dem Anwender spontan oder nach einer Weile zu einer ungewünschten physiologischen Reaktion, wie z.B. einer Hautrötung oder der Ausbildung eines Juckreizes kommt.

Bevorzugt werden die erfindungsgemäßen Zusammensetzungen nach einem Verfahren hergestellt, bei dem
a) ein Lipid in einem kurzkettigen aliphatischen Monoalkohol, bevorzugt Ethanol, gelöst wird, wobei eine Lipidlösung erhalten wird,
b) ein positiv geladenes Alkyl-Trimoniumsalz in der Lipidlösung aus a) gelöst wird,
c) wenigstens ein lipophiler direktziehender Haarfarbstoff in die Lösung aus b) gegeben wird
d) die Lösung aus c) unter Rühren in eine Wasserphase gegeben wird, wobei eine Emulsion erhalten wird,
e) der pH-Wert der Emulsion durch Zugabe einer Base, bevorzugt Natronlauge, auf einen physiologischen pH-Wert von pH 6 bis 7 eingestellt wird, und
f) optional die Emulsion durch Hochdruckhomogenisation bei einem Druck von bis zu 1000 bar bis 1500 bar homogenisiert wird.

Unter einem kurzkettigen aliphatischen Monoalkohol ist dabei ein Monoalkohol mit einer Kettenlänge von C1 bis C4 zu verstehen.

Alternativ können erfindungsgemäße Zusammensetzungen nach einem Verfahren hergestellt werden, bei dem
a) ein Lipid in einem kurzkettigen aliphatischen Monoalkohol, bevorzugt Ethanol, gelöst wird, wobei eine Lipidlösung erhalten wird,
b) ein positiv geladenes Alkyl-Trimoniumsalz in der Lipidlösung von a) gelöst wird,
c) wenigstens ein hydrophiler direktziehender Haarfarbstoff in eine Wasserphase gegeben wird
d) die Lösung aus b) unter Rühren in die Wasserphase aus c) gegeben wird, wobei eine Emulsion erhalten wird,
e) der pH-Wert der Emulsion durch Zugabe einer Base, bevorzugt Natronlauge, auf einen physiologischen pH-Wert von pH 6 bis 7 eingestellt wird und
f) optional die Emulsion durch Hochdruckhomogenisation bei einem Druck von bis zu 1000 bar bis 1500 bar homogenisiert wird.

Bei den erfindungsgemäßen Zusammensetzungen, die sowohl einen lipophilen als auch einen hydrophilen Haarfarbstoff enthalten, ist eine Kombination der beiden oben beschriebenen Verfahren besonders bevorzugt.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

Des Weiteren wird darauf hingewiesen, dass es für den Fachmann selbstverständlich ist, dass die nachfolgenden Ausführungsbeispiele lediglich dazu dienen, zwei mögliche Ausführungsformen der vorliegenden Erfindung beispielhaft anzugeben. Der Fachmann wird daher ohne weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende, explizite Darstellung sämtlicher denkbarer Ausführungsformen innerhalb der beanspruchten Erfindung wird hier nur der Kürze und der Lesbarkeit der Beschreibung wegen verzichtet.

### Ausführungsbeispiel 1 (Haarfarbstoff in der Lipidmembran)

Ausführungsbeispiel 1 betrifft Lipidvesikel mit der folgenden Zusammensetzung:
10,00 Gew.-% Phospholipide (Lecithin)
12,00 Gew.-% Ethanol
4,00 Gew.-% Behentrimoniumchlorid
1,00 Gew.-% direktziehender Haarfarbstoff
Ad 100 Gew.-% Wasser, VE

Um die Lipidvesikel gemäß Ausführungsbeispiel 1 herzustellen, wurde das Lecithin in Ethanol vollständig solubilisiert. In diese Lösung wurden Behentrimoniumchlorid und der Farbstoff unter Rühren eingearbeitet und vollständig gelöst. Nach einem Homogenisationsverfahren wurde diese Lipidphase in Wasser eingearbeitet.

Die Teilchengröße der nach diesem Verfahren hergestellten kationischen Lipidvesikel wurde mit Hilfe der Photonenkorrelationsspektroskopie (PCS, Zetamaster S, Malvern Instruments Ltd., UK) kontrolliert, wobei der Herstellungsprozess nach Erreichen einer mittleren Teilchengröße mit einem Durchmesser von etwa 100 bis 350 nm beendet wurde.

### Ausführungsbeispiel 2 (Haarfarbstoff im Vesikelinnenraum)

Ausführungsbeispiel 2 betrifft Lipidvesikel mit der folgenden Zusammensetzung:
7,00 Gew.-% Phospholipide (Lecithin)
12,00 Gew.-% Ethanol
3,00 Gew.-% Behentrimoniumchlorid
5,00 Gew.-% direktziehender Haarfarbstoff Ad 100 Gew.-% Wasser, VE

Um die Lipidvesikel gemäß Ausführungsbeispiel 2 herzustellen, wurde das Lecithin in Ethanol vollständig solubilisiert. In diese Lösung wurden nacheinander Behentrimoniumchlorid sowie der Farbstoff unter Rühren eingearbeitet. In diese Lipidphase wurde schließlich die angegebene Wassermenge homogen eingearbeitet und dabei mit der Lipidphase gemischt. Anschließend wurde diese Emulsion mit Hilfe der Hochdruckhomogenisation homogenisiert.

Die Teilchengröße der nach diesem Verfahren hergestellten kationischen Lipidvesikel wurde mit Hilfe der Photonenkorrelationsspektroskopie (PCS, Zetamaster S, Malvern Instruments Ltd., UK) kontrolliert, wobei der Herstellungsprozess nach Erreichen einer mittleren Teilchengröße mit einem Durchmesser von etwa 100 bis 400 nm beendet wurde.

## Patentansprüche

1. Kosmetische Zusammensetzung zum Färben der Haare mit wenigstens einem direktziehenden Haarfarbstoff und mit einem Trägersystem für den wenigstens einen direktziehenden Haarfarbstoff, wobei das Trägersystem Lipidvesikel mit einer oder mehreren Lipidmembran(en) umfasst, **dadurch gekennzeichnet, dass** der wenigstens eine direktziehende Haarfarbstoff in den Lipidvesikeln enthalten ist und die Lipidvesikel eine positive Oberflächenladung aufweisen, wobei die positive Oberflächenladung dadurch bewirkt wird, dass die Lipidvesikel in der/den Lipidmembran/en zusätzlich zu den Lipiden, aus denen die Vesikel aufgebaut sind, positiv geladene Moleküle als Ladungsgeber aufweisen, wobei diese Ladungsgeber aus Alkyl-Trimoniumsalzen der Formel 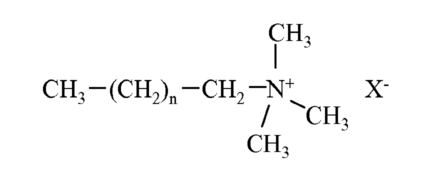 ausgewählt sind, wobei
n eine Zahl von 18 bis 28 und
X- ein anorganisches oder organisches Anion ist.

2. Zusammensetzung gemäß Anspruch 1, **dadurch gekennzeichnet, dass** X- Bromid, Chlorid, Fluorid, Iodid, Saccharinat, Tosylat oder Methosulfat ist.

3. Zusammensetzung gemäß einem der Ansprüche 1 oder 2, **dadurch gekennzeichnet, dass** der Anteil der Ladungsgeber bezogen auf die gesamte Zusammensetzung 0,01 bis 10 Gew.-% beträgt.

4. Zusammensetzung gemäß einem der vorangehenden Ansprüche, **dadurch gekennzeichnet, dass** das Zetapotential der Lipidvesikel von 1 bis 200 mV beträgt.

5. Zusammensetzung gemäß einem der vorangehenden Ansprüche, **dadurch gekennzeichnet, dass** die Lipidvesikel eine Teilchengröße von 50 bis 1000 nm haben.

6. Zusammensetzung gemäß einem der vorangehenden Ansprüche, **dadurch gekennzeichnet, dass** die Lipide, aus denen die Vesikel aufgebaut sind, unter Ceramiden, Phospholipiden, Glycosphingolipiden und/oder Di-Acylglycosiden ausgewählt sind.

7. Zusammensetzung gemäß einem der vorangehenden Ansprüche, **dadurch gekennzeichnet, dass** der Anteil der Lipide bezogen auf die gesamte Zusammensetzung 1 bis 20 Gew.-% beträgt.

8. Zusammensetzung gemäß einem der vorangehenden Ansprüche, **dadurch gekennzeichnet, dass** der wenigstens eine direktziehende Haarfarbstoff ausgewählt ist unter lipophilen und hydrophilen physiologisch aktiven Substanzen, die aus natürlichen Quellen isoliert oder chemisch oder biotechnologisch hergestellt wurden, oder Kombinationen davon.

9. Zusammensetzung gemäß einem der vorangehenden Ansprüche, **dadurch gekennzeichnet, dass** der Anteil des wenigstens einen direktziehenden Haarfarbstoffs bezogen auf die gesamte Zusammensetzung 0,01 bis 40 Gew.-% beträgt.

10. Zusammensetzung gemäß einem der Ansprüche 1 bis 9, **dadurch gekennzeichnet, dass** sie zusätzlich einen oder mehrere Hilfsstoffe umfaßt.

11. Verwendung einer Zusammensetzung gemäß einem der Ansprüche 1 bis 10 zur Herstellung einer kosmetischen Formulierung zum Färben der Haare.

12. Kosmetische Formulierung, die eine Zusammensetzung gemäß einem der Ansprüche 1 bis 10 enthält.

13. Verwendung gemäß Anspruch 11 oder Formulierung gemäß Anspruch 12, **dadurch gekennzeichnet, dass** die Formulierung für die topische Applikation ist.

14. Verfahren zur Herstellung von Zusammensetzungen gemäß einem der Ansprüche 1 bis 10, **dadurch gekennzeichnet, dass**
a) ein Lipid in einem kurzkettigen aliphatischen Monoalkohol gelöst wird, wobei eine Lipidlösung erhalten wird,
b) ein positiv geladenes Alkyl-Trimoniumsalz, in der Lipidlösung von a) gelöst wird,
c) wenigstens ein lipophiler direktziehender Haarfarbstoff in die Lösung aus b) gegeben wird,
d) die Lösung aus c) unter Rühren in eine Wasserphase gegeben wird, wobei eine Emulsion erhalten wird,
e) der pH-Wert der Emulsion durch Zugabe einer Baseauf einen physiologischen pH-Wert von pH 6 bis 7 eingestellt wird, und
f) optional die Emulsion durch Hochdruckhomogenisation bei einem Druck von bis zu 1000 bar bis 1500 bar homogenisiert wird.

15. Verfahren zur Herstellung von Zusammensetzungen gemäß einem der Ansprüche 1 bis 10, **dadurch gekennzeichnet, dass**
a) ein Lipid in einem kurzkettigen aliphatischen Monoalkohol gelöst wird, wobei eine Lipidlösung erhalten wird,
b) ein positiv geladenes Alkyl-Trimoniumsalz in der Lipidlösung von a) gelöst wird,
c) wenigstens ein hydrophiler direktziehender Haarfarbstoff in eine Wasserphase gegeben wird
d) die Lösung aus b) unter Rühren in die Wasserphase aus c) gegeben wird, wobei eine Emulsion erhalten wird,
e) der pH-Wert der Emulsion durch Zugabe einer Base auf einen physiologischen pH-Wert von pH 6 bis 7 eingestellt wird, und
f) optional die Emulsion durch Hochdruckhomogenisation bei einem Druck von bis zu 1000 bar bis 1500 bar homogenisiert wird.
